# EUROPEAN PATENT SPECIFICATION

(11) **EP 1 123 508 B1**
(45) Date of publication and mention of the grant of the patent: **01.09.2004**
(21) Application number: 99970737.5
(22) Date of filing: 20.10.1999
(51) Int. Cl.: G01N 33/49, G01N 33/566, G01N 33/86, G01N 33/88

(54) **METHOD FOR MEASURING CELLULAR ADHESION**
VERFAHREN ZUR MESSUNG DER ZELLULÄREN ADHÄSION
PROCEDE DE MESURE DE L'ADHERENCE CELLULAIRE

(30) Priority: 20.10.1998 AU PP660798
(43) Date of publication of application: 16.08.2001
(73) Proprietor: MONASH UNIVERSITY, Clayton Victoria 3168 (AU)
(72) Inventor: DOPHEIDE, Sacha, Marie, Mont Albert, VIC 3127 (AU); COOKE, Brian, Mark, Hawthorn, VIC 3122 (AU); JACKSON, Shaun, Phillip, North Balwyn, VIC 3104 (AU)
(74) Representative: Harding, Charles Thomas
(86) International application number: PCT/AU1999/000901
(87) International publication number: WO 2000/023802

(56) References cited:
- US-A- 4 936 674
- US-A- 5 523 238
- CARROLL L. RAMOS ET AL: "Quantitative measurement of cell-cell adhesion under flow conditions" METHODS IN MOLECULAR MEDICINE, vol. 18, 1998, pages 507-519, XP001029269
- MAMMEN EBERHARD F. ET AL: "PFA-100 system: a new method for assessment of platelets" SEMINARS IN THROMBOSIS AND HEMOSTASIS, vol. 24, no. 2, 1998, pages 195-202, XP001029142
- JACKSON S.P. ET AL: "Ectoenzyme regulation of plaletet adhesion and thrombus formation under flow" BLOOD, vol. 92, no. 10 suppl. 1, 15 November 1998 (1998-11-15), page 345A XP001029255
- MULVIHILL J. ET AL.: 'Thrombin stimulated platelet accumulation on protein coated glass capillaries: role of adhesive platelet alpha-granule proteins' THROMBOSIS AND HAEMOSTATIS vol. 62, no. 3, 04 November 1989, pages 989 - 995, XP001029326
- MULVIHILL J. ET AL.: 'The use of monoclonal antibodies to human platelet membrane glycoprotein IIb-IIIa to quantitate platelet deposition on artificial surfaces' THROMBOSIS AND HAEMOSTATIS vol. 58, no. 2, 04 August 1987, pages 724 - 731, XP001029332
- HO M. ET AL.: 'Characterization of Plasmodium falciarum infected erythrocyte and P selectin interaction under flow conditions' BLOOD vol. 91, no. 12, 15 June 1998, pages 4803 - 4809, XP002907341
- TSUJI S. ET AL.: 'Real-time analysis of mural thrombus formation in various platelet aggregation disorders: distinct shear-dependent roles of platelet receptors and adhesive proteins under flow' BLOOD vol. 94, no. 3, 01 August 1999, pages 968 - 975, XP001029054
- YEO L. ET AL.: 'Role of P selectin and leukocyte activatin in polymorphonuclear cell adhesion to surface adherent activated platelets under physiologic shear conditions (an injury vessel wall model)' BLOOD vol. 83, no. 9, 01 May 1994, pages 2498 - 2507, XP001029333
- MOROI M. ET AL.: 'Analysis of the involvement of the von Willebrand Factor - glycoprotein Ib interaction in platelet adhesion to a collagen coated surface under flow conditions' BLOOD vol. 90, no. 11, 01 December 1997, pages 4413 - 4424, XP001029052

## Description

### FIELD OF THE INVENTION

The present invention relates generally to a method for determining and quantifying cellular adhesive phenomena. More particularly, the present invention contemplates a flow-based system for determining the binding capability of platelets or their progenitors or other cells in biological samples which may be either fully or semi-automated. The method of the present invention is particularly useful in assessing individuals for abnormalities in platelet activity or function, such as those occurring in thrombosis, heart disease, stroke or other vascular diseases.

### BACKGROUND OF THE INVENTION

Platelets are specialised adhesive cells which play a central role in haemostatic processes such as blood clotting. If the endothelial cells which line a blood vessel are damaged, platelets will rapidly adhere and aggregate at the site of injury, forming the primary haemostatic plug. In addition to their role in the arrest of bleeding, platelets also play a central role in the formation of life-threatening arterial thrombi. Studies of patients suffering from heart attacks and stroke have demonstrated that these diseases are precipitated by the rupture of an atherosclerotic plaque, exposing elements in the vessel wall capable of activating platelets. The adhesion of platelets to exposed subendothelial components, combined with the release of chemical mediators capable of inducing platelet aggregation and vasoconstriction, results in the generation of life-threatening thrombi.

Platelet adhesion processes are critically dependent on the interaction of platelet surface receptors with specific adhesive proteins present in the damaged blood vessel wall. Two of these proteins, von Willebrand factor (vWf) and collagen, play a critical role in mediating the initial adhesion of platelets to the vessel wall. Von Willebrand factor binds to two major receptors on the platelet surface, the glycoprotein (GP) Ib/V/IX complex and GPIIb/IIIa. The initial interaction between platelets and vWf is mediated by the vWf-GPIb/V/IX interaction. This is a transient adhesion event that not only supports platelet rolling but also transmits signals required for GPIIb/IIIa activation. This latter receptor binds to a distinct site on the vWf molecule and stabilises platelet adhesion to the site of vascular injury, particularly under conditions of high shear stress. Collagen also binds two distinct receptors on the platelet surface, GPIa/IIa and GPVI. Recent studies suggest that both of these receptors are required for efficient platelet adhesion on collagen, with the latter receptor (GPVI) playing an important role in collagen-induced signal transduction. A number of other receptors on the platelet surface, including integrin α₅β₁ (a fibronectin receptor), integrin α₆ β₁ (a laminin receptor) and integrin α₅β₃ (a vitronectin receptor) may also be involved in the initial adhesion process.

The reason for such a large number of adhesion receptors on the platelet surface has not been clearly defined. One possibility is that the platelet may employ distinct adhesion mechanisms at different sites of the vascular tree. For example, it is well established that under conditions of high shear stress, such as those encountered in the microcirculation or in stenotic arteries, platelet adhesion is critically dependent on the binding of vWf to both GPIbN/IX and GPIIb/IIIa. Under these conditions, collagen, fibrinogen, fibronectin, vitronectin or laminin cannot support stable platelet adhesion in the absence of vWf. However, under lower shear conditions, such as in veins or large arteries, vWf is not essential for stable platelet adhesion, and one or more of these latter substrates may mediate stable platelet adhesion.

Mulvihill *et al* (Thrombosis and Haemostasis 1989 vol. 62 p989) disclose the role of adhesive platelet α-granule proteins in thrombin stimulated platelet accumulation on protein coated glass capillaries. Mulvihill *et al* (Thrombosis and Haemostasis 1987 vol. 58 p724) disclose the use of monoclonal antibodies to human platelet membrane glycoprotein IIb-IIIa to quantitate platelet deposition on artificial surfaces.

At present there are no routine clinical tests which assess the adhesion process for platelets and other cells over a range of shear stresses and on a range of different adhesive surfaces. Given the critical role for platelet adhesion in many disorders including haemostasis and thrombosis, there is an important clinical need for the development of simple, rapid and reliable diagnostic tests which adequately assess the adhesive function of platelets.

One system currently commercially available for analysis of platelet function (PFA-100, manufactured and marketed by DADE International) is designed to measure primary platelet dependent haemostasis in citrated whole blood *in vitro*, and consists of a microprocessor-controlled instrument and a disposable test cartridge. The test cartridge contains a reservoir for citrated whole blood and a capillary surmounted by a cup containing a biologically active membrane with a central aperture. The membrane is coated with fibrillar type 1 equine tendon collagen. Additionally, either epinephrine (adrenaline) or ADP is present on the membrane to enhance platelet activation. The analyser aspirates whole blood through the capillary into the cup where it comes into contact with the membrane and then passes through the aperture. In response to stimulation by collagen, ADP or adrenaline, and high shear stress (∼5,000-6,000s⁻¹), platelets adhere and aggregate at the membrane surface at the area surrounding the aperture. A platelet plug ultimately occludes the aperture and the blood flow stops. The time required to obtain occlusion of the aperture is defined as the closure time.

In work leading up to the current invention, the inventors have developed a flow-based method for measuring the adhesion or binding capability of platelets and other cells in biological samples. The method allows for copying or mimicking a range of conditions such as flow-rate and wall shear stress typical of those that occur *in vivo*.

### SUMMARY OF THE INVENTION

In one aspect, the present invention provides a diagnostic method for detection in an individual of the presence of or risk of developing a condition or disorder involving cellular abnormalities, comprising:
i determining the binding capacity of cells in a biological sample from the individual to an adhesive substrate by passing said biological sample in a laminar flow over an adhesive substrate immobilised on a solid support under defined flow conditions and for a time sufficient to enable cells from the biological sample to bind to the adhesive substrate, and subsequently determining the number of cells bound to the adhesive substrate to indicate the binding capacity of said cells; and
ii comparing the determined binding capacity of said cells in the biological sample with a predetermined standard binding capacity for such cells, wherein variation of the determined binding capacity from the predetermined standard binding capacity is indicative of the presence of or risk of developing a condition or disorder involving cellular abnormalities in the individual.

In a particular embodiment, the present invention enables the measurement of the propensity for platelets to form an adhesive clot under conditions that mimic normal homeostasis. It can, therefore, be used as a diagnostic tool to determine cardiovascular risk in a human or animal, particularly the risk of complications associated with excessive bleeding and/or clotting events such as polycythemia, heart disease, stroke, transient ischaemia or peripheral vascular disease.

In a further aspect, the present invention provides a method for determining the modulating effect of a substance on the binding capacity of cells in a biological sample, from an individual, comprising
i passing said biological sample in the presence of said substance over an adhesive substrate immobilised on a solid support under defined flow conditions and for a time sufficient to enable cells from the biological sample to bind to the adhesive substrate, and subsequently detecting cells bound to the adhesive substrate; and
ii comparing the results obtained in step (i) with the result obtained when step (i) is performed in the absence of said substance.

In yet another aspect, the present invention provides a system for detection in an individual of the presence of or risk of developing a condition or disorder involving cellular abnormalites, comprising:
i. a solid support having an adhesive substrate immobilised thereon;
ii. means for passing a biological sample from the individual in laminar flow over said adhesive substrate under defined flow conditions and for a time sufficient to enable cells from the biological sample to bind to the adhesive substrate; and
iii. detection means to determine the number of said cells bound to the adhesive substrate.

### DETAILED DESCRIPTION OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The term "biological sample" as used herein is intended to include any sample which contains cells putatively exhibiting binding capacity including, but not limited to, processed and unprocessed biological samples such as whole blood (native or anticoagulated), plasma, platelets, platelets (washed and resuspended in isotonic buffer), lymph, white cells, red blood cells, tissue extract or biopsy material.

Preferably, the cells comprise platelets or progenitors thereof.

The term "individual" as used herein is intended to include a healthy individual as well as an individual with known or suspected cardiovascular or other related condition or disorder.

The term "adhesive substrate" is used herein in its broadest sense to include any ligand to which a cell in the biological sample may interact to an extent resulting in permanent or temporary binding of the cell to the ligand. Adhesive substrates include receptors for cell surface ligands, ligands for cell surface receptors, and immunoglobulin molecules capable of interacting with cell surface antigens or epitopes, immobilised cells with interaction capacity for cells in the sample, as well as specific or non-specific aggregation substrates.

In a preferred embodiment, the adhesive substrate is suitable for the adhesion or binding of platelets or progenitors thereof and includes *inter alia* molecules which interact with cell surface receptors such as vWf, fibrinogen, collagen, vitronectin, laminin or fibronectin.

The term "solid support" as used herein includes any device, apparatus, tube (including microcapillary tube), chamber, filter, pipe or slide suitable for immobilising an adhesive substrate. The solid support may be for example a solid material such as a polymer, glass, polycarbonate, polyvinyl chloride, metal or cellulose. Alternatively, the solid support may be a surface of biological material such as cells or tissue or any other biological substrate suitable for immobilising an adhesive substrate.

The term "determining the number of cells" includes subjecting immobilised cells from the biological sample to a process which is capable of detecting the immobilised cells. Conveniently, a marker is selected on the immobilised cell surface or in the membrane or cytosol of the cell, and the marker detected. Where necessary, the immobilised cell may be wholly or partially disrupted in order to gain access to the marker. Alternatively, the cell may need to be permeabilised to allow intracellular marker detection. The marker may be, for example, an antigen, enzyme, receptor, ligand for a receptor or any other molecule capable of being used as a detection marker.

In one preferred embodiment, the marker is one which is suitable for detection of platelets or progenitors thereof bound to the adhesive substrate. Such markers and include *inter alia* lactate dehydrogenase (LDH), platelet factor 4, P-selectin, thromboxane B2 or β-thromboglobulin.

In a particularly preferred embodiment, the marker is detected by spectrophotometric means, such as in the detection of platelet LDH.

The term "flow rate" is also referred to throughout the specification by the equivalent terms: "perfusion rate", "shear rate" or "wall shear stress". The biological sample may be passed over the immobilised adhesive substrate using any flow regulating means, such as a single speed pump, a variable speed pump, a syringe pump or gravitational forces. Regulation of the flow rate may be achieved by any suitable method, such as variation of pump speed.

Flow rate is defined as millilitres of fluid per minute. Shear is a consequence of the relative parallel motion of fluid planes during flow, such that in a vessel, the velocity of fluid near the wall is lower than towards the centre. This difference in flow rate between concentric layers of fluid creates a "shearing" effect. Shear is defined as either shear rate or shear stress. Shear rate is expressed as cm/s per cm (or inverse second-s⁻¹). Shear stress is force per unit area (expressed as Dyn/cm² or Pascals) and is equivalent to shear rate x viscosity.

Typical flow rates contemplated herein cover the range required to develop those proposed to exist in the vasculature *in vivo.* Typically, shear rates range from about 20 to about 20,000s⁻¹, more particularly from about 30 to about 3000s⁻¹.

In the method of the present invention as broadly described above, the detection of cells such as platelets bound to the adhesive substrate may be achieved by disrupting the bound cells and detecting the disrupted cells.

The term "disrupting" as used herein is intended to include any process or method for washing, lysing, permeabilising or otherwise altering the cells. It is further contemplated that the disruption of the cells bound to the adhesive substrate can be performed with a suitable agent such as a lysis buffer or detergent. Disruption of the cells is useful for quantification of certain markers.

Determination of the binding capacity of cells, particularly platelets or progenitors thereof, in accordance with the present invention provides a diagnostic method for detection of individuals with or at risk of developing conditions or disorders involving cellular abnormalities, in particular abnormalities involving platelets or progenitors thereof. Table 1 refers to many conditions where accurate assessment of platelet function will assist the diagnosis and appropriate management of the treatment of individuals. Furthermore, ongoing monitoring of platelet function will also assist in assessing the response of an individual to treatment.

In particular, the method of the present invention provides a diagnostic method for determining the risk of an individual developing a blood clot. The risk of an individual developing a blood clot may be determined by making a comparison between different groups of individuals. For example, a comparison may be made of blood samples from normal healthy individuals and blood samples from patients with a risk of developing a blood clot, by measuring and comparing adhesion of platelets from the blood samples of each group over a standardised, specified period of time at a specified flow rate and temperature.

Currently there are no specific tests which provide information on the likelihood of an individual developing an arterial blood clot and the consequent risk of heart attack or stroke. Increased risk of heart attack and stroke is currently assessed indirectly by measuring the level of cholesterol and blood glucose, coupled with clinical assessment in addition to heart disease and stroke, abnormalities of platelet function are associated with many other disorders (see Table 1). In all these conditions, accurate assessment of platelet function will significantly assist the diagnosis and appropriate management of the patient. Ongoing monitoring of platelet function will also assist in assessing a patient's response to treatment.

**Table 1:**

| **Importance of platelet activity in various diseases** | | |
|---|---|---|
| **Hyperactivity has a primary role** | **Hyperactivity has important contributory role** | **Reduced activity has primary role** |
| 1. Heart diseases, including heart attack, sudden death, stable and unstable angina 2. Diseases of the brain, including stroke, transient ischaemic attacks (TlAs). 3. Diseases of peripheral blood vessels, including large and small peripheral vessel disease. | 4. Spread of cancer 5. Inflammatory diseases including glomerulonephritis (inflammation of the kidney), acute respiratory distress syndrome | 6. Inherited bleeding disorders 7. Bleeding complications associated with Kidney failure 8. Bleeding complications associated with Liver failure 9. Drug-related bleeding 10. Bleeding complicating surgery 11. Major blood transfusion |

Clinical conditions contemplated by the method of the present invention include, but are not limited to, full cardiovascular risk assessment in otherwise healthy individuals; assessment of patients who have suffered a thrombotic event; monitoring of the effectiveness of prescribed anti-platelet therapy; assessment of bleeding or clotting risk in patients scheduled for major surgery; assessment of the clotting risk profile in patients at high risk of cardiovascular disease, including those with diabetes mellitus, hypertension, high blood cholesterol, strong family history of clotting, smokers and those with identifiable thrombosis markers; assessment of clotting risk in patients with peripheral vascular disease; and investigation of the profile of patients with bleeding disorders.

As described above, in another aspect, the invention provides a diagnostic method for detection in an individual of the presence of or risk of developing a condition or disorder involving cellular abnormalities, comprising:
i obtaining a biological sample from the individual;
ii determining the binding capacity of cells in the biological sample to an adhesive substrate by passing said biological sample in a laminar flow over an adhesive substrate immobilised on a solid support under defined flow conditions and for a time sufficient to enable cells from the biological sample to bind to the adhesive substrate, and subsequently determining the number of said cells bound to the adhesive substrate to indicate the binding capacity of said cells; and
iii comparing the determined binding capacity of cells in the biological sample with a predetermined standard binding capacity for such cells, wherein variation of the determined binding capacity from the predetermined standard cbinding capacity is indicative of the presence of or risk of developing a condition or disorder involving cellular abnormalities in the individual.

In work leading to the present invention, it has been determined that, in a control population of healthy individuals, the mean platelet count, as determined by the method and device as described herein at a shear rate of 600s⁻¹ using vWf or collagen as the stationary phase adhesive substrate, falls within the range 170-450 LDH (U/L). Individuals with a platelet count less than 170 LDH (U/L) are considered to be at risk from complications associated with excessive bleeding, and those individuals with a platelet content greater than 450 LDH (U/L) are considered to have an increased risk of developing complications associated with inappropriate clot formation.

In a further aspect, the present invention provides a system for detection in an individual of the presence of or risk of developing a condition or disorder involving cellular abnormalites, comprising:
i. a solid support having an adhesive substrate immobilised thereon;
ii. means for passing a biological sample from the individual in laminar flow over said adhesive substrate under defined flow conditions and for a time sufficient to enable cells from the biological sample to bind to the adhesive substrate; and
iii. detection means to determine the number of said cells bound to the adhesive substrate.

Preferably, the solid support is a microcapillary or other tube and the adhesive substrate is immobilised on the intemal surface thereof. Preferably also, the biological sample is perfused or caused to flow through the microcapillary tube by means of a pump such as a syringe pump.

The present invention also provides a method for determining the modulating effect of a substances on the binding capacity of cells in a biological sample from an individual, comprising firstly passing said biological sample in the presence of said substance over an adhesive substrate immobilised on a solid support under defined flow conditions and for a time sufficient to enable cells from the biological sample to bind to the adhesive substrate, and subsequently detecting cells bound to the adhesive substrate; and then comparing the result obtained in step (i) with the result when step (i) is performed in the absence of said substance.

As used herein, the term "substance" is used in its broadest sense to encompass a single compound or mixture of compounds. The term also includes both synthetic and natural substances; including biological materials such as antibodies, hormones, other proteins or polypeptides, and the like.

The substance may be, for example, a known anti-platelet agent. Alternatively, the substance may be a substance which is to be screened for its modulating effect on platelets or progenitors thereof, or other cells.

The term "modulation" is used herein to refer to any effect which the substance has on the binding capacity of cells such as platelets or progenitors thereof to the adhesive substrate. Accordingly, the term includes both enhancement and inhibition of this binding capacity.

In its preferred embodiments, the present invention provides a flow-based assay that allows the quantitative assessment of platelet adhesion over a wide range of shear stresses. With this system, adhesion assays are performed in microcapillary tubes that are coated with adhesive proteins present in the normal damaged blood vessel wall. The microcapillary tubes are coated with various adhesive substrates, such as von Willebrand factor (vWf), fibrinogen or collagen. Other relevant substrates include vitronectin, laminin or fibronectin or any other adhesive surface that has the capacity to support platelet adhesion. Platelets in whole blood (native or anticoagulated), plasma, or washed platelets (resuspended in an isotonic buffer), are perfused through the microcapillary tubes at defined flow rates achieving wall shear rates over the range (20-20,000s⁻¹) proposed to exist in the vasculature *in vivo*. Flow (and hence shear stress), is precisely controlled using a syringe pump. Perfusion times can be varied from a few seconds up to 60 minutes. Following perfusion of platelets through the microcapillary tubes, unbound cells are removed by washing at the same shear stress, red cells lysed (using 1% ammonium oxalate or any other solution that preferentially lyses red blood cells), and the remaining adherent platelets lysed with detergent. The platelet lysates are assayed for a platelet marker (i.e. lactate dehydrogenase, platelet factor 4, P-selectin, thromboxane B2, β-thromboglobulin, or any other marker that accurately predicts the number of platelets adherent to the microcapillary tube). In situations where the marker is specific to platelets (i.e. platelet factor 4, β-thromboglobulin), the red cell lysis step can be omitted. The number of platelets adherent to the adhesive substrate coating the microcapillary tubes may then be determined by comparing the level of this marker against a predetermined standard which has been obtained using known amounts of platelets.

Using normal healthy blood donors, it has been demonstrated that this assay is a reliable and reproducible method for quantitating platelet adhesion. The coefficient of variation (CV) on a vWf matrix, using a single blood donor, is 13% whereas the CV between donors is 19%, all within acceptable range for such clinical tests as defined by the International Committee for Standardisation in Haematology. Assays can be performed on anti-coagulated whole blood (using citrate, heparin or PPACK) or with washed platelets for up to four hours after collection of blood. Time course studies have demonstrated that there is a linear increase in the number of platelets accumulating onto the coated microcapillary tubes over the first 5 minutes of perfusion time. Matrix concentration is also a major determinant of platelet adhesion, with maximal adhesion at a vWf coating concentration of 100 µg/ml. The effect of shear rate on platelet adhesion onto a vWf matrix has been demonstrated, and increasing the shear from 750s⁻¹ to 3,000s⁻¹ leads to a corresponding increase in the number of platelets adherent to the vWf matrix.

An important observation with the assay of this invention is that there is a significant difference between platelets from males and females in terms of adhesion onto a vWf matrix, and studies indicate that 50-80% more platelets from mates adhere onto the matrix than platelets from females under identical assay conditions. Despite intense investigation, a sex-specific difference in platelet reactivity has never been detected using a variety of platelet functional assays. This sex-specific difference in platelet adhesiveness assumes importance in light of the fact that males are 6-7 times more likely to suffer from a heart attack between the age of 25-55 than women. These studies also demonstrate that females with cardiovascular disease appear to have significantly higher levels of platelet adhesion than healthy female controls. It is well established that platelets from patients with cardiovascular disease have a heightened reactivity to exogenous stimuli and will spontaneously aggregate *in vitro*.

Adhesion studies have also been performed on several other adhesive matrices, including bovine vWf, fibrinogen and type 1 fibrillar collagen. As with human vWf, platelet adhesion on these surfaces is reproducible with CVs between 10-15% for a single donor. As with human vWf, the matrix concentration plays an important role in determining the number of platelets adherent to the surface of each matrix. The effect of increasing shear on platelet adhesion to fibrinogen and collagen has been examined. In contrast to vWf, fibrinogen is only able to support stable platelet adhesion at shear forces below 150s⁻¹. In contrast, collagen is able to support stable platelet adhesion at shear rates as high as 3,000s⁻¹. Platelet adhesion onto collagen at shear rates >750s⁻¹ is critically dependent on the vWf-GPlb interaction. The assay also provides a useful way of examining the efficacy of anti-platelet drugs. For example, pretreating platelets with the anti-GPIIb/IIIa drug, ReoPro (5 µg/ml), leads to a 90% reduction in platelet adhesion onto vWf.

The diagnostic system of the present invention has a number of significant differences from the DADE PFA-100 system currently available, as discussed above. These include:
1. In the present system, the flow is laminar, well defined, and fixed throughout each individual assay. In the PFA-100 system, flow (and therefore shear stress) is variable, depending on the extent of aperture occlusion;
2. In the present system, assays can be performed over a wide range of shear rates (high and low, physiological and pathological). In the PFA-100 system, all assays are performed at high shear rates (5,000-6,000s⁻¹);
3. In the present system, platelet adhesion to a range of physiologically-relevant surfaces can be assessed. Furthermore, soluble agonists, such as ADP or adrenaline, do not need to be added to the adhesive surface to enhance platelet activation. The PFA-100 system is an occlusion-based system that requires strong platelet activation, thus limiting the types of adhesive surfaces available for the assay. The formation of an occlusive thrombus requires type 1 fibrillar collagen (which is the most potent thrombogenic surface *in vitro*), high shear stress (to enhance platelet thrombus formation) and soluble agonists, such as ADP or adrenaline (to potentiate platelet activation); and
4. In the present system, the number of platelets adherent to the matrix can be determined directly by measuring a platelet marker in the whole cell lysates. The PFA-100 system measures platelet accumulation indirectly by monitoring the occlusion time.

The present invention is exemplified herein with respect to human cells. However, this is done with the understanding that the present invention extends to the cells of all animals including, livestock animals (e.g. sheep, cows, horses, donkeys), laboratory test animals (e.g. rats, guinea pigs, rabbits, hamsters), companion animals (e.g. dogs, cats), captive wild animals (e.g. kangaroos, deer, foxes) and poultry birds (e.g. chickens, ducks, bantams, pheasants).

Further features of the present invention are more fully described in the following detailed description and examples. It is to be understood, however, that this detailed description and examples are included solely for the purposes of exemplifying the present invention. It should not be understood in any way as a restriction on the broad description of the invention as set out above.

In the Figures:

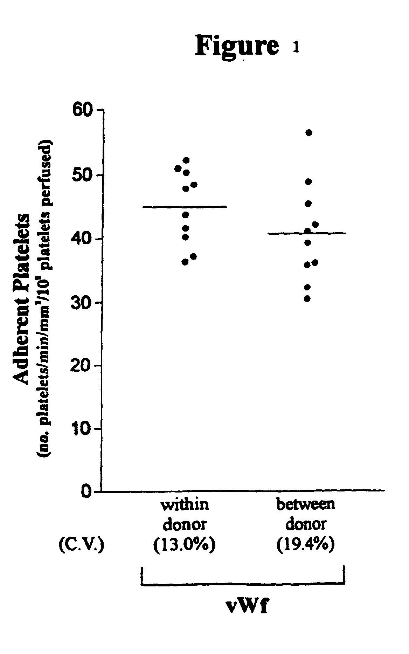

- **Figure 1**: is a graphical representation of the variability of platelet adhesion onto a vWf matrix. The coefficient of variation is shown within each group.
- **Figure 2**: is a graphical representation of the effect of different anticoagulants on platelet adhesion onto a vWf matrix.
- **Figure 3**: is a graphical representation of the time course of platelet adhesion under flow.
- **Figure 4**: is a graphical representation of the effect of increasing shear on platelet adhesion on vWf.
- **Figure 5**: is a graphical representation highlighting the differences in platelet adhesion between male and female blood donors.
- **Figure 6**: is a graphical representation of the effect on platelet adhesion using blood collected from female donors with a history of vascular disease.
- **Figure 7**: is a graphical representation of the effect of increasing shear on platelet adhesion to a fibrinogen matrix.
- **Figure 8**: is a graphical representation of the effect of increasing shear on platelet adhesion to a collagen matrix.
- **Figure 9**: is a graphical representation of the dependence of GPIb for platelet adhesion to a collagen matrix at high shear.
- **Figure 10**: is a graphical representation of the effect of anti-platelet drugs on platelet adhesion onto a vWf matrix.
- **Figure 11**: shows the normal variability of platelet adhesion onto a vWf matrix in the platelet adhesion assay.
- **Figure 12**: shows the normal variability of platelet adhesion onto a collagen matrix in the platelet adhesion assay.
- **Figure 13**: shows the normal variability of platelet adhesion onto a collagen matrix at high shear in the platelet adhesion assay.
- **Figure 14**: shows a comparison, in the platelet adhesion assay of the present invention, of platelet adhesion onto collagen-coated microcapillaries after wet or dry storage of the microcapillaries for up to 8 weeks.
- **Figure 15**: shows levels of platelet adhesion to a vWf matrix using blood collected from donors with no complications, bleeding history or clotting history.
- **Figure 16**: shows levels of platelet adhesion to collagen matrix using blood collected from donors with no complications, bleeding history or clotting history.
- **Figure 17**: shows the effect of Aggrastat at three different concentrations and ReoPro at a single concentration on platelet adhesion onto vWf-coated microcapillary tubes at a shear rate of 600s^{-1.}
- **Figure 18**: is a diagrammatic representation of a device which may be used for determination of the adhesion or binding capacities of platelets in a blood sample or cell suspension in accordance with the present invention.
- **Figure 19**: is a schematic diagram of a semi-automated device in accordance with the present invention.

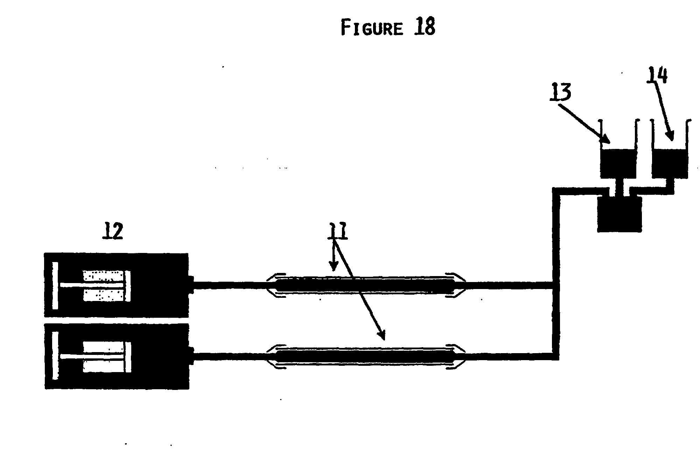

Referring firstly to Figure 18, the device shown diagrammatically comprises a number of microcapillary tubes 11 which are coated internally with an adhesive substrate, such as vWf or collagen, and which are in fluid connection with syringe pumps 12 to enable liquids to be perfused through tubes 11 under defined flow conditions and times. Tubes 11 are also connected to sample reservoir 13 and wash buffer reservoir 14.

In use of the device shown in Figure 18, a blood sample or cell suspension from a patient is introduced into sample reservoir 13, and is then pulled through the capillary tubes 11 at a predetermined flow rate by syringe pumps 12. Wash buffer from reservoir 14 is then washed through the tubes 11 and the tubes are removed. Subsequently, red blood cells adhering to the tubes may be lysed as described above and the lysate discarded. Platelets adhering to the tubes may then be lysed, and the lysate collected and assayed for LDH content as previously described.

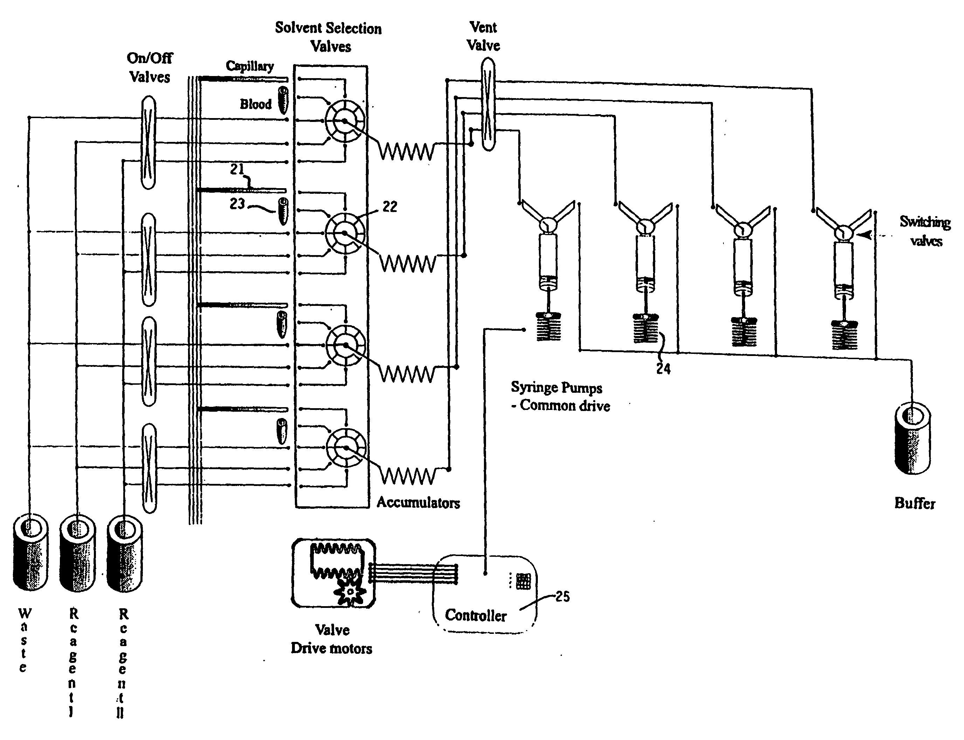

Figure 19 shows schematically one example of a semi-automated device in accordance with the present invention which enables analysis of a number of individual blood samples. In this device, each intemally-coated microcapillary tube 21 is connected to a selection valve 22 that may be selected to enable perfusion of a blood sample 23, or of wash buffer or lysis reagents I or II through the microcapillary tube 21. The flow rate of a blood sample through each tube 21 is controlled by a syringe pump 24, which in turn is controlled by controller 25. Specific software programs may be used to ensure perfusion of blood samples at strictly defined, predetermined flow rates, and that the various washing procedures and subsequent lysis of cells such as red blood cells and/or platelets which adhere to each microcapillary tube 21 are automated under control by the software programs. The lysis sample obtained in each microcapillary tube 21 is analysed by spectrophotometry as previously described.

### EXAMPLE 1

### Variation in platelet adhesion on a vWf-matrix under flow

The variation in platelet adhesion was examined using the method of the present invention. For these studies, microcapillary tubes were coated with vWf (100µg/ml) for 2 hours at room temperature, then blocked with 25% v/v heat-inactivated human serum for 60 minutes at room temperature. Analysis of variation within one donor was performed by perfusing washed platelets from a single donor through 10 separate vWf-coated microcapillary tubes on the same day. In separate experiments, platelets obtained from 10 different donors were perfused through vWf-coated microcapillary tubes on different days.

Washed platelets obtained from one donor (within donor) or different donors (between donors) were perfused through vWf-coated microcapillary tubes for 5 minutes at 150s⁻¹. Non-adherent platelets were removed by washing at 150s⁻¹ for 10 minutes, and the number of platelets adherent to the matrix quantitated and expressed as the no. adherent platelets/min/mm²/10 ⁸ platelets perfused. The results from 10 separate assays are presented.

The coefficient of variation (CV) of a single donor was 13.0%, whereas the CV between donors was 19.4%. Both CVs are within acceptable ranges for clinical tests.

### EXAMPLE 2

### Effect of anticoagulant on platelet adhesion under flow

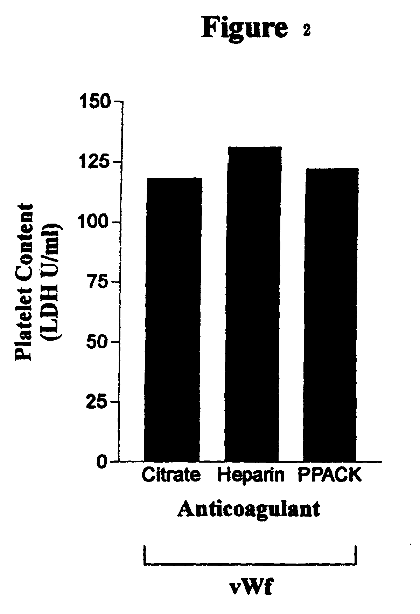

Given that a number of different anticoagulants are used for blood collection in clinical practice, the effects of three different anticoagulants (citrate, heparin and PPACK) on platelet adhesion was investigated using the method of the present invention. Whole blood collected in the presence of citrate (0.38% w/v tri-sodium citrate), low molecular weight heparin (10U/ml) or PPACK (40µM), was perfused through vWf-coated microcapillary tubes for 5 minutes at 150s⁻¹. Non-adherent cells were removed from the tube by washing for 10 minutes at 150s⁻¹, and adherent erythrocytes lysed through treatment with 1% w/v ammonium oxalate. Adherent platelets and thrombi were lysed with 1% v/v Triton X-100 and collected for subsequent LDH analysis. LDH (U/ml) levels in the whole cell lysates were quantitated by spectrophotometry. The results presented in Figure 2 demonstrate that platelet accumulation onto the vWf surface was unaffected by the type of anticoagulant used.

### EXAMPLE 3

### Time course for platelet adhesion under flow

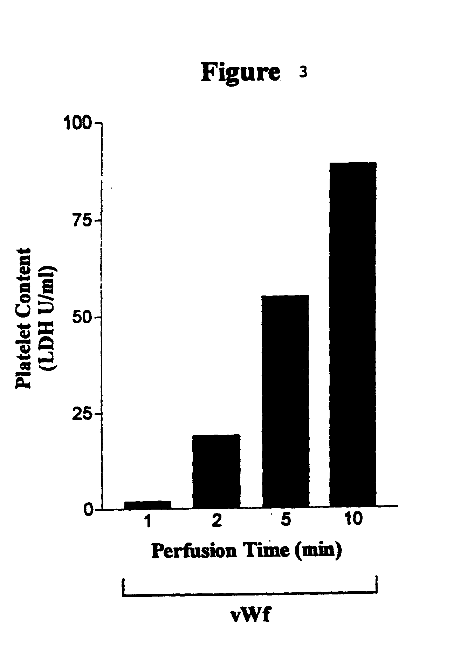

The time course for platelet adhesion on vWf under flow was examined. Citrated whole blood was perfused through vWf-coated microcapillary tubes for 1, 2, 5 or 10 minutes at 150s⁻¹. Non-adherent cells were removed from the tube by washing for 10 minutes at 150s⁻¹, and cells lysed as described in Example 2. The extent of platelet adhesion was quantitated indirectly by measuring platelet LDH as described in Example 2. Figure 3 demonstrates that with increasing perfusion times, there is a corresponding increase in platelet accumulation onto the vWf surface.

### EXAMPLE 4

### Effect of increasing shear on platelet adhesion on vWf under flow

It is well established that platelet adhesion occurs over a wide range of shear stresses *in vivo*, and that a number of subendothelial proteins and platelet surface receptors are required to mediate this process. Under conditions of high shear, such as those encountered in arterioles or stenotic vessels, platelet adhesion to subendothelial-bound vWf is essential. However at lower shear conditions other adhesive proteins, such as collagen or fibronectin, can mediate platelet adhesion independent of vWf.

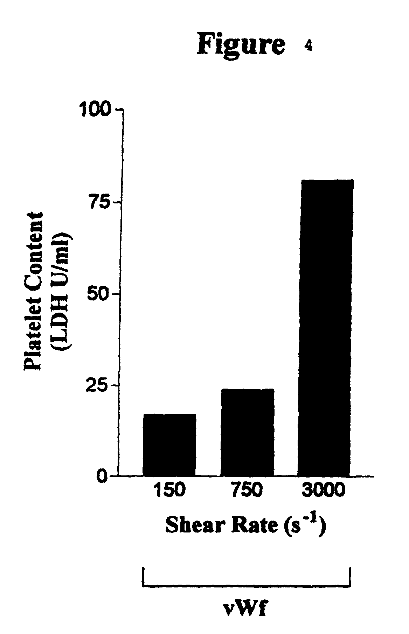

To investigate the role of vWf in mediating platelet adhesion at different shear condition, equal volumes (8 mls) of citrated whole blood was perfused through vWf-coated microcapillary tubes at flow rates of 150, 750 and 3000s⁻¹. Non-adherent cells were removed from the tube by washing for 10 minutes at 150s⁻¹, and adherent erythrocytes lysed through treatment with 1% w/v ammonium oxalate. Adherent platelets were lysed with 1% v/v Triton X-100 and collected for subsequent LDH analysis. The extent of platelet adhesion was quantitated indirectly by measuring platelet LDH as described in Example 2. The results as shown in Figure 4, demonstrate that with increasing shear stress there is a corresponding increase in the number of adherent platelets.

### EXAMPLE 5

### Sex specific differences in platelet adhesion under flow

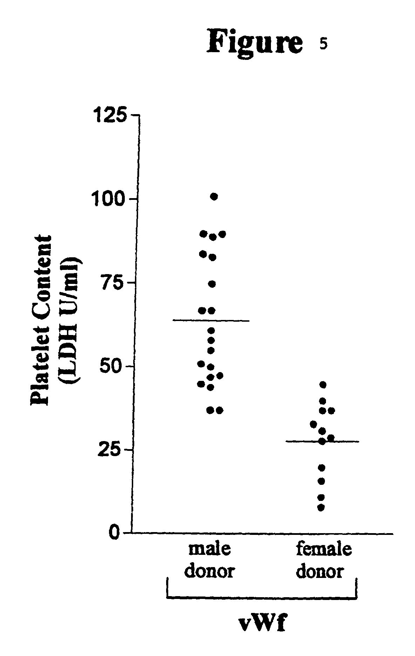

Previous studies have demonstrated that males are 6-7 times more likely to suffer from a heart attack between the ages of 25-55 than women. The following test was performed to investigate whether there is a sex-specific difference in platelet adhesion under flow. Citrated whole blood collected from healthy male and female donors between the ages of 25-55, who had no previous history of cardiovascular disease, was perfused through vWf-coated microcapillary tubes for 10 minutes at 150s⁻¹. Following washing for 10 minutes at 150s¹ to remove non-adherent cells, adherent erythrocytes were lysed with 1% w/v ammonium oxalate. Adherent platelets and thrombi were subsequently lysed from the surface with 1% v/v Triton X-100 and collected for LDH analysis by measuring platelet LDH as described in Example 2. The results as shown in Figure 5 indicate that 50-80% more platelets from male donors accumulate onto the vWf surface as compared to females.

In contrast, blood collected from female donors with a previous history of heart and/or cardiovascular disease resulted in approximately two-fold increase in platelet adhesion onto the vWf surface.

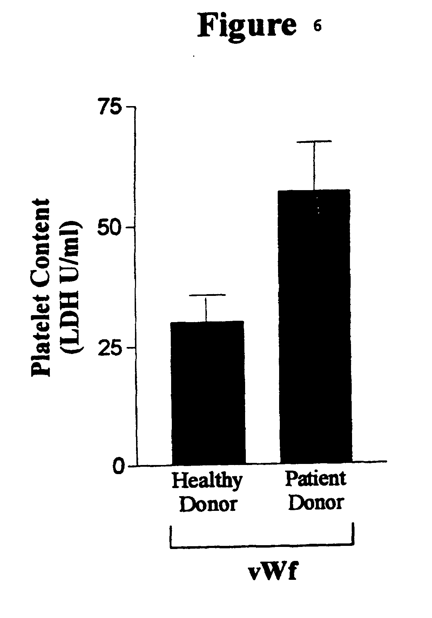

Citrated whole blood collected from either normal female donors, or those with a history of cardiovascular disease (acute coronary syndromes or cerebrovascular accidents) was perfused through vWf-coated microcapillary tubes at 150s⁻¹ for 10 minutes. Non-adherent cells were removed by washing for 10 minutes at 150s⁻¹, and the extent of platelet adhesion quantitated indirectly by measuring platelet LDH as described in Example 2. The results are shown in Figure 6.

### EXAMPLE 6

### Effect of increasing shear on platelet adhesion to fibrinogen and collagen substrates under flow

To investigate the effect of wall shear rate on platelet adhesion onto a fibrinogen or collagen substrate equivalent volumes of citrated whole blood were perfused through collagen- or fibrinogen-coated microcapillary tubes at various shear rates.

Washed platelets were perfused through fibrinogen-coated microcapillary tubes at shear rates of 30, 75, 150 and 750s⁻¹ for 5 minutes. Non-adherent platelets were removed by washing at the same shear rate for 10 minutes, and the number of platelets adherent to the matrix quantitated and expressed as the no. adherent platelets/min/mm²/10⁸ platelets perfused.

Equal volumes (8mls) of citrated whole blood was perfused through collagen-coated microcapillary tubes at shear rates of 150, 750 and 3000s⁻¹ for 5 minutes. Non-adherent cells were removed by washing for 10 minutes at 150s⁻¹, and the extent of platelet adhesion quantitated indirectly by measuring platelet LDH as described in Example 2.

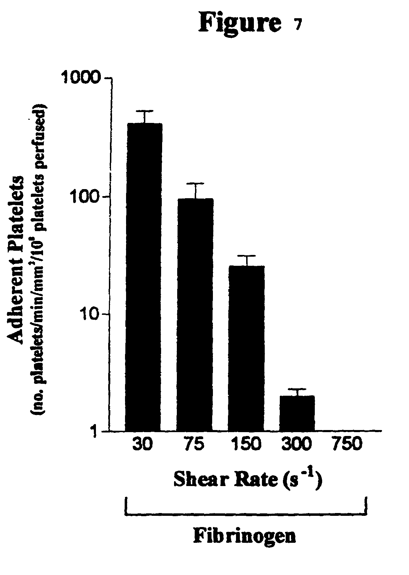

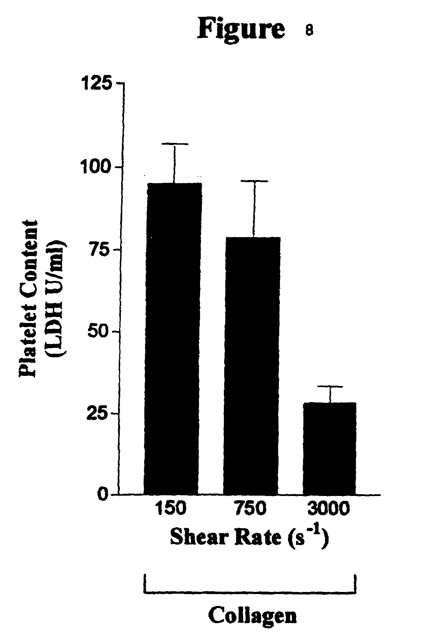

Figure 7 demonstrates that platelet adhesion onto a fibrinogen substrate was maximal at low shear rates (<150s⁻¹) with a corresponding decrease in platelet adhesion with higher shear, such that at 750s⁻¹ no platelets adhered onto the adhesive substrate. As demonstrated in Figure 8, a similar trend was observed with platelet adhesion onto a collagen substrate, however, this substrate was able to support platelet adhesion at high shear rates (>3,000s⁻¹).

### EXAMPLE 7

### GPIb -dependent Platelet Adhesion to Collagen at High Shear Rates

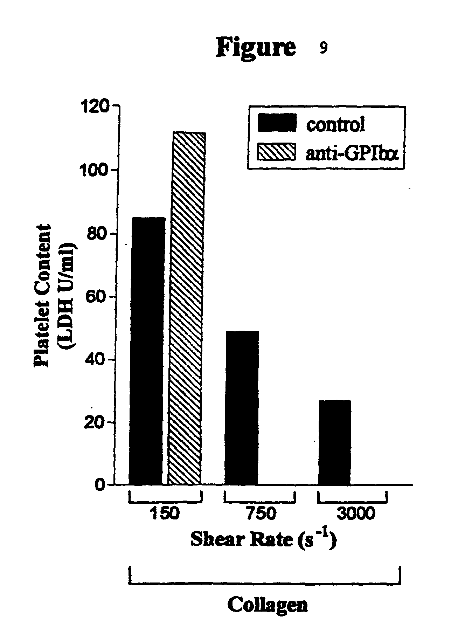

Platelet adhesion to either vWf or collagen at high shear rates has previously been shown to be dependent on the GPIb-vWf interaction. Platelet adhesion to collagen under high shear requires deposition of plasma-derived vWf onto the immobilised collagen matrix. Once immobilised, vWf supports platelet adhesion through its interaction with GPIb. To investigate the role of GPIb in supporting platelet adhesion to collagen using the method of the present invention, citrated-whole blood was treated with the anti-GPIb antibody, AK2, for 30 minutes at room temperature. Equal volumes (8 mls) of untreated and anti-GPIb treated blood were then perfused through collagen-coated microcapillary tubes at 150, 750, and 3000s⁻¹. Non-adherent cells were removed by washing the tubes for 10 minutes at 150s^{-1,} and the platelet LDH levels quantitated as described in Example 2 to determine the extent of platelet adhesion. As demonstrated in Figure 9, platelet adhesion to collagen was abolished at shear rates above 750s⁻¹ when ligand binding to GPIb was prevented.

### EXAMPLE 8

### Effect of anti-platelet drugs on platelet adhesion under flow

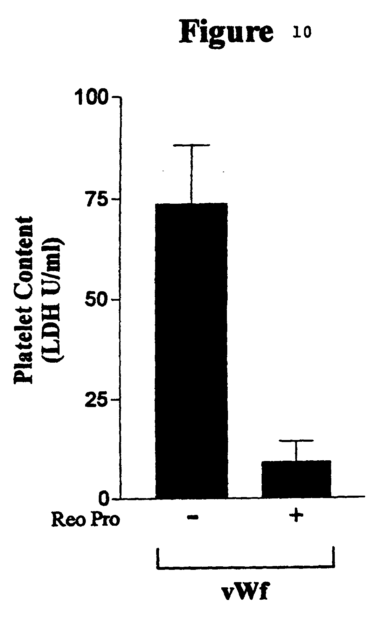

The effect of the anti-platelet drug, ReoPro (7E3), on platelet adhesion on vWf under flow was investigated. Citrated-whole blood pretreated with ReoPro (5µg/ml) for 10 minutes at room temperature, was perfused through vWf-coated microcapillary tubes for 10 minutes at 150s⁻¹. Non-adherent cells were removed by washing for 10 minutes at 150s⁻¹, and any adherent erythrocytes lysed through treatment with 1% ammonium oxalate. Adherent platelets were then lysed through addition of 1% Triton X-100, and LDH (U/ml) levels analysed through spectrophotometry. As demonstrated in Figure 10, treatment of blood with ReoPro had a dramatic effect on platelet adhesion to the vWf matrix, reducing this by approximately 90%.

### EXAMPLE 9

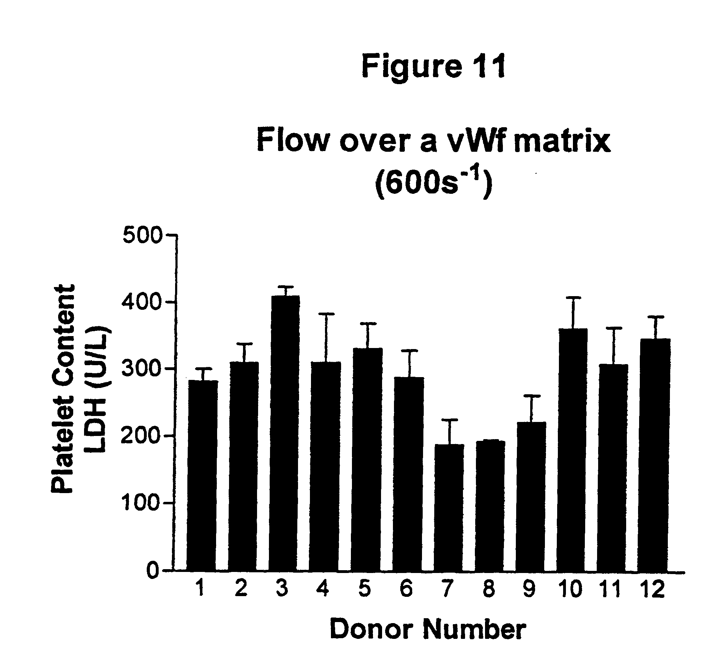

The reproducibility of platelet adhesion in the platelet adhesion assay of the present invention is demonstrated in Figures 11, 12 and 13 for different surface coatings and shear conditions.

Whole blood anticoagulated with citrate was perfused through vWf-coated microcapillary tubes at 600s⁻¹ for 2 minutes. Non-adherent cells were removed by washing with Tyrode's buffer, and any adherent erythrocytes lysed through treatment with 1% ammonium oxalate. Adherent platelets were subsequently lysed with 1% Triton X-100, and the LDH (U/L) levels in the whole cell lysates quantitated by spectrophotometry. The results from 12 different normal donors are presented in Figure 8. For each donor, blood was perfused through four separate vWf-coated microcapillary tubes on the same day. The mean +2SD is shown for each donor.

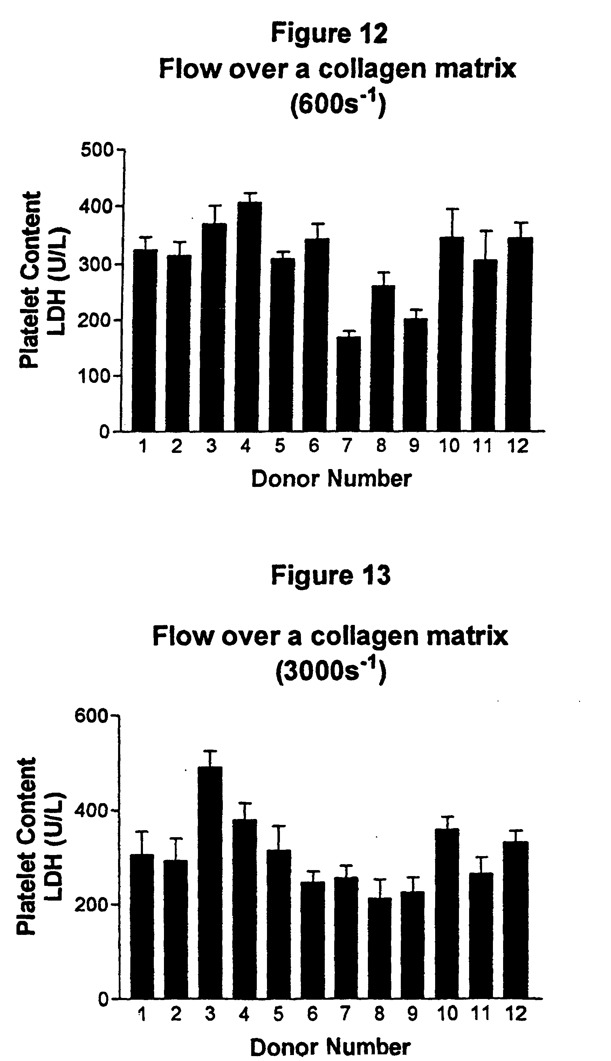

Whole blood anticoagulated with citrate was perfused through collagen-coated microcapillary tubes at 600s⁻¹ for 2 minutes. Non-adherent cells were removed by washing with Tyrode's buffer, and the extent of platelet adhesion quantitated indirectly by measuring platelet LDH as described above. The results from 12 different normal donors are presented in Figure 12. For each donor, blood was perfused through four separate collagen-coated microcapillary tubes on the same day. The mean +2SD is shown for each donor.

Whole blood anticoagulated with citrate was perfused through collagen-coated microcapillary tubes at 3000s⁻¹ for 1 minute. Non-adherent cells were removed by washing, and the extent of platelet adhesion quantitated indirectly by measuring platelet LDH as described above. The results from 12 different normal donors are presented in Figure 13. For each donor, blood was perfused through four separate collagen-coated microcapillary tubes on the same day. The mean +2SD is shown for each donor.

### EXAMPLE 10

Levels of platelet adhesion were determined using collagen-coated microcapillaries after wet or dry storage of the microcapillaries for up to 8 weeks.

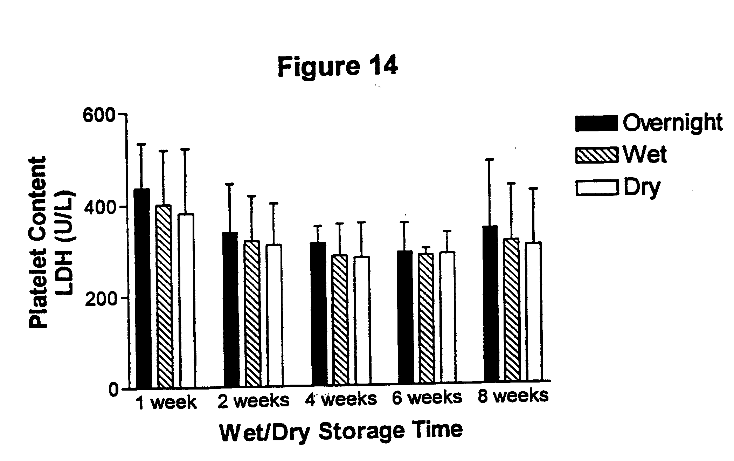

Microcapillaries coated with collagen under standard conditions (over night at 4°C) were compared with coated microcapillaries stored wet at 4°C for 1-8 weeks or dry for 1-8 weeks. Whole blood anticoagulated with citrate was perfused through the collagen-coated microcapillary tubes at 3000s⁻¹ for 1 minute. Non-adherent cells were removed by washing, and the extent of platelet adhesion quantitated indirectly by measuring platelet LDH as described in Example 9. The results from 5 different experiments are presented in Figure 14. In each experiment, blood was perfused through four of each of the types of collagen-coated microcapillary tubes on the same day. The meant +2SD is shown for each coating in each experiment.

### EXAMPLE 11

Levels of platelet adhesion were determined in the platelet adhesion assay of the present invention, within a group of patients with myetoproliferative disorders, subgrouped on the basis of an uncomplicated clinical history (defined as no clinically documented clotting or bleeding episode - this definition does not exclude subclinical thrombotic disease), bleeding complications or clotting complications.

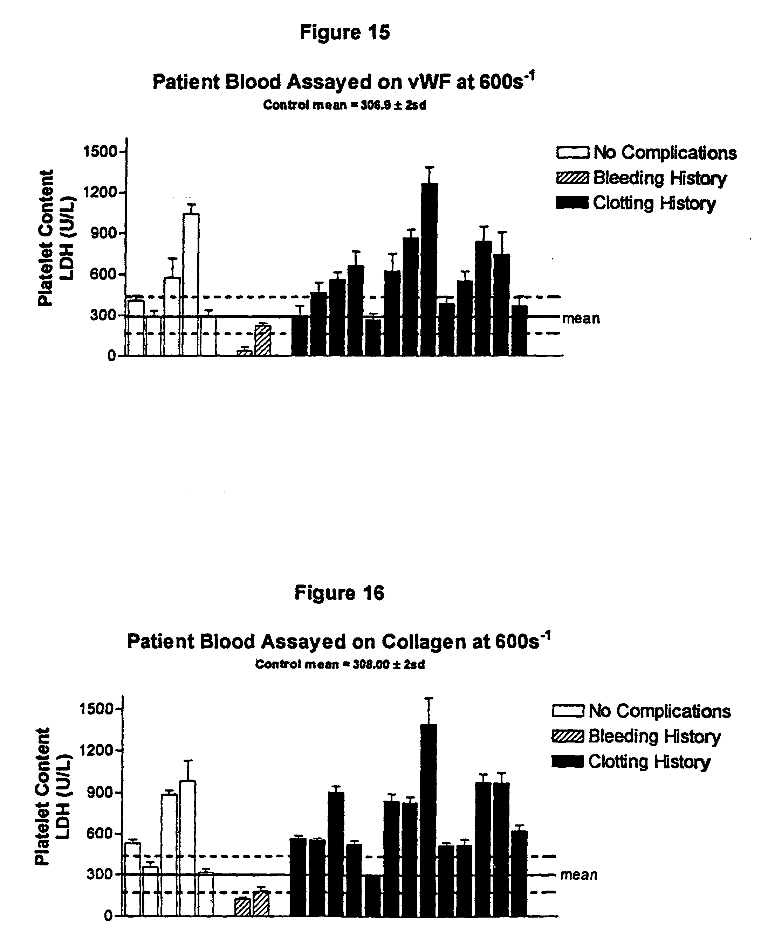

Whole blood, anticoagulated with citrate was perfused through microcapillary tubes coated with collagen or vWf as indicated at a shear rate of 600s⁻¹. Non-adherent cells were removed by washing, and the extent of platelet adhesion quantitated indirectly by measuring platelet LDH as described in Example 9. The results of these assays are shown in Figures 15 and 16. In each experiment, the patient blood sample was perfused through four separate microcapillary tubes on the same day. The mean +2SD is shown for each patient.

### EXAMPLE 12

The effect of pretreatment of whole blood with anti-platelet agents. Aggrastat or ReoPro, on platelet adhesion was determined in the platelet adhesion assay of the present invention.

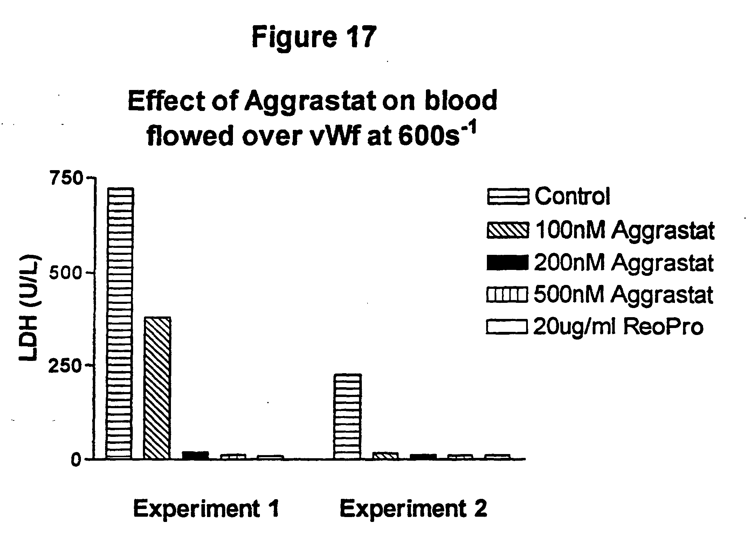

Whole blood was incubated with 100, 200 or 500 nM Aggrastat, or 20 µg/ml ReoPro for 10 minutes at 37°C before perfusing through a vWf-coated microcapillary tube at 600s⁻¹ for 2 minutes. Non-adherent cells were removed by washing, and the extent of platelet adhesion quantitated indirectly by measuring platelet LDH as described in Example 9. The results of these assays are shown in Figure 17.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

## Claims

1. A diagnostic method for detection in an individual of the presence of or risk of developing a condition or disorder involving cellular abnormalities, comprising:
i. determining the binding capacity of cells in a biological sample from the individual by passing said biological sample in laminar flow over an adhesive substrate immobilised on the internal surface of a microcapillary tube under defined flow conditions and for a time sufficient to enable cells from the biological sample to bind to the adhesive substrate, and subsequently determining the number of said cells bound to the adhesive substrate by disrupting the bound cells and then detecting a marker on the cell surface or in the membrane or cytosol of said cells to indicate the binding capacity of said cells; and
ii. comparing the determined binding capacity of said cells in the biological sample with a predetermined standard binding capacity for such cells, wherein variation of the determined binding capacity from the predetermined standard binding capacity is indicative of the presence of or risk of developing a condition or disorder involving cellular abnormalities in the individual.

2. A diagnostic method according to claim 1, wherein the biological sample is selected from the group consisting of whole native blood, whole anticoagulated blood, plasma, platelets, platelets washed and resuspended in isotonic buffer, lymph, white cells, red blood cells, tissue extract or biopsy material.

3. A diagnostic method according to claim 1, wherein the cells comprise platelets or progenitors thereof.

4. A diagnostic method according to claim 1, wherein the adhesive substrate is selected from the group consisting of receptors for cell surface ligands, ligands for cell surface receptors, and immunoglobulin molecules capable of interacting with cell surface antigens or epitopes, immobilised cells with interaction capacity for cells in the sample.

5. A diagnostic method according to claim 4, wherein the adhesive substrate binds platelets or progenitors thereof and is selected from the group consisting of vWf, fibrinogen, collagen, vitronectin, laminin or fibronectin.

6. A diagnostic method according to claim 1, wherein the defined flow conditions comprise a shear rate in the range of from about 20 to about 20,000s⁻¹, more particularly from about 30 to about 3,000s⁻¹.

7. A diagnostic method according to claim 1, wherein the cells are platelets or progenitors thereof, and the marker is selected from the lactate dehydrogenase (LDH), platelet factor 4, P-selectin, thromboxane B2 or β-thromboglobulin.

8. A diagnostic method according to claim 7, wherein the marker is LDH and is detected by spectrographic means.

9. A diagnostic method according to claim 1, wherein the condition or disorder is development of a blood clot.

10. A diagnostic method according to claim 1, wherein said method is used in full cardiovascular risk assessment in otherwise healthy individuals; assessment of patients who have suffered a thrombotic event; monitoring of the effectiveness of prescribed anti-platelet therapy; assessment of bleeding or clotting risk in patients scheduled for major surgery; assessment of the clotting risk profile in patients at high risk of cardiovascular disease; assessment of clotting risk in patients with peripheral vascular disease; or investigation of the profile of patients with bleeding disorders.

11. An apparatus for detection in one or more individuals of the presence of or risk of developing a condition or disorder involving cellular abnormalites, comprising:
i a control device;
ii. a plurality of microcapillary tubes, each having an adhesive substrate immobilised on the internal surface thereof;
iii. a pump controlled by said control device for passing a biological sample from the or each individual through each of said microcapillary tubes in laminar flow over said adhesive substrate under defined flow conditions and for a time sufficient to enable cells from the biological sample to bind to the adhesive substrate; and
iv. detection means controlled by said control device to determine the number of said cells bound to the adhesive substrate in each of said microcapillary tubes by disrupting the bound cells and then detecting a marker on the cell surface or in the membrane or cytosol of said cells;
wherein said detection means comprises a spectrophotometer.

12. An apparatus according to claim 11, further comprising:
v. means for comparing the determined binding capacity of said cells with a predetermined standard binding capacity for such cells.

13. An apparatus according to claim 11, wherein the adhesive substrate binds platelets or progenitors thereof and is selected from the group consisting of vWf, fibrinogen, collagen, vitronectin, laminin or fibronectin.

14. A method for determining the modulating effect of a substance on the binding capacity of cells in a biological sample from an individual, comprising
i. passing said biological sample in the presence of said substance in laminar flow over an adhesive substrate immobilised on the internal surface of a microcapillary tube under defined flow conditions and for a time sufficient to enable cells from the biological sample to bind to the adhesive substrate, and subsequently determining the number of said cells bound to the adhesive substrate disrupting the bound cells and then by detecting a marker on the cell surface or in the membrane or cytosol of said cells to indicate the binding capacity of said cells in the presence of said substance; and
ii. comparing the result obtained in step (i) with the result obtained when step (i) is performed in the absence of said substance.

15. A diagnostic method for detection in one or more individuals of the presence of or risk of developing a condition or disorder involving cellular abnormalities, comprising:
i. determining the binding capacity of cells in a plurality of biological samples from the or each individual in a system comprising:
(a) a control device;
(b) a plurality of microcapillary tubes, each having an adhesive substrate immobilised on the internal surface thereof;
(c) a pump controlled by said control device for passing a biological sample through each of said microcapillary tubes in laminar flow over said adhesive substrate under defined flow conditions and for a time sufficient to enable cells from the biological sample to bind to the adhesive substrate; and
(d) detection means controlled by said control device to determine the number of said cells bound to the adhesive substrate in each of said microcapillary tubes by disrupting the bound cells and then detecting a marker on the cell surface or in the membrane or cytosol of said cells; and
ii. comparing the determined binding capacity of said cells in each biological sample with a predetermined standard binding capacity for such cells, wherein variation of the determined binding capacity from the predetermined standard binding capacity is indicative of the presence of or risk of developing a condition or disorder involving cellular abnormalities in the individual providing said biological sample.

16. A method according to claim 15, wherein said system further comprises:
(e) means for comparing the determined binding capacity of said cells in each biological sample with a predetermined standard binding capacity for such cells.

17. A diagnostic method according to claim 15, wherein the biological samples are selected from the group consisting of whole native blood, whole anticoagulated blood, plasma, platelets, platelets washed and resuspended in isotonic buffer, lymph, white cells, red blood cells, tissue extract or biopsy material.

18. A diagnostic method according to claim 15, wherein the cells comprise platelets or progenitors thereof.

19. A diagnostic method according to claim 15, wherein the adhesive substrate is selected from the group consisting of receptors for cell surface ligands, ligands for cell surface receptors, and immunoglobulin molecules capable of interacting with cell surface antigens or epitopes, immobilised cells with interaction capacity for cells in the sample.

20. A diagnostic method according to claim 19, wherein the adhesive substrate binds platelets or progenitors thereof and is selected from the group consisting of vWf, fibrinogen, collagen, vitronectin, laminin or fibronectin.

21. A diagnostic method according to claim 15, wherein the defined flow conditions comprise a shear rate in the range of from about 20 to about 20,000s⁻¹, more particularly from about 30 to about 3,000s⁻¹.

22. A diagnostic method according to claim 15, wherein the cells are platelets or progenitors thereof, and the marker is selected from the lactate dehydrogenase (LDH), platelet factor 4, P-selectin, thromboxane B2 or β-thromboglobulin.

23. A diagnostic method according to claim 22, wherein the marker is LDH and is detected by spectrographic means.

24. A diagnostic method according to claim 15, wherein the condition or disorder is development of a blood clot.

25. A diagnostic method according to claim 15, wherein said method is used in full cardiovascular risk assessment in otherwise healthy individuals; assessment of patients who have suffered a thrombotic event; monitoring of the effectiveness of prescribed anti-platelet therapy; assessment of bleeding or clotting risk in patients scheduled for major surgery; assessment of the clotting risk profile in patients at high risk of cardiovascular disease; assessment of clotting risk in patients with peripheral vascular disease; or investigation of the profile of patients with bleeding disorders.

## Patentansprüche

1. Diagnoseverfahren zur Bestimmung des Vorliegens oder des Risikos zur Entwicklung eines Zustands oder einer Funktionsstörung, die mit zellulären Abnormalitäten verknüpft ist, bei einem Individuum, umfassend:
i. das Bestimmen der Bindungskapazität von Zellen in einer biologischen Probe des Individuums durch Führen dieser biologischen Probe in laminarer Strömung über ein adhäsives Substrat, das an der inneren Oberfläche eines Mikrokapillarröhrchens immobilisiert ist unter definierten Strömungsbedingungen und einer Zeitdauer, die ausreicht, die es den Zellen der biologischen Probe ermöglicht, an das adhäsive Substrat zu binden, und anschließendes Bestimmen der Anzahl der Zellen, die an das adhäsive Substrat gebunden sind, indem die gebundenen Zellen zerstört werden und anschließend ein Marker an der Zelloberfläche oder in der Membran oder dem Zytosol dieser Zellen detektiert wird, um die Bindungskapazität dieser Zellen anzugeben; und
ii. Vergleichen der ermittelten Bindungskapazität dieser Zellen in der biologischen Probe mit einer zuvor bestimmten Standardbindungskapazität für solche Zellen, wobei eine Abweichung der ermittelten Bindungskapazität von der zuvor ermittelten Standardbindungskapazität auf das Vorliegen oder das Risiko zur Entwicklung eines Zustands oder einer Funktionsstörung, die mit zellulären Abnormalitäten verknüpft ist, bei dem Individuum hinweist.

2. Diagnoseverfahren nach Anspruch 1, worin die biologische Probe ausgewählt ist aus der Gruppe, bestehend aus natürlichem Vollblut, vollständig nicht gerinnbarem Blut, Plasma, Plättchen, Plättchen, die gewaschen und in isotonischem Puffer resuspendiert worden sind, Lymphe, weißen Blutkörperchen, roten Blutkörperchen, Gewebsextrakt oder Biopsie-Material.

3. Diagnoseverfahren nach Anspruch 1, worin die Zellen Plättchen oder deren Vorgänger umfassen.

4. Diagnoseverfahren nach Anspruch 1, worin das adhäsive Substrat ausgewählt ist aus der Gruppe, bestehend aus Rezeptoren für Zelloberflächenliganden, Liganden für Zelloberflächenrezeptoren und Immunoglobulin-Molekülen, die befähigt sind mit Zelloberflächenantigenen oder Epitopen in Wechselwirkung zu treten, immobilisierten Zellen, die mit Zellen in der Probe in Interaktion treten können.

5. Diagnoseverfahren nach Anspruch 4, worin das adhäsive Substrat Plättchen oder deren Vorgänger bindet und ausgewählt ist aus der Gruppe, bestehend aus vWf, Fibrinogen, Kollagen, Vitronectin, Laminin oder Fibronectin.

6. Diagnoseverfahren nach Anspruch 1, worin die definierten Strömungsbedingungen eine Scherrate in dem Bereich von etwa 20 bis zu etwa 20.000 s⁻¹, insbesondere von etwa 30 zu etwa 3.000 s⁻¹, umfassen.

7. Diagnoseverfahren nach Anspruch 1, worin die Zellen Plättchen oder deren Vorgänger sind und der Marker ausgewählt ist aus Laktatdehydrogenase (LDH), Plättchenfaktor 4, P-Selektin, Thromboxan B2 oder β-Thromboglobulin.

8. Diagnoseverfahren nach Anspruch 7, worin der Marker LDH ist und über spektrographische Mittel detektiert wird.

9. Diagnoseverfahren nach Anspruch 1, worin der Zustand oder die Fehlfunktion die Entwicklung eines Blutgerinnsels ist.

10. Diagnoseverfahren nach Anspruch 1, worin diese Methode bei der Beurteilung des kardiovaskulären Risikos bei sonst gesunden Individuen verwendet wird; der Beurteilung von Patienten, die einen thrombotischen Vorfall erlitten haben; bei der Überwachung der Wirksamkeit der zuvor beschriebenen Anti-Plättchentherapie; zur Beurteilung des Blutungs- oder Koagulationsrisikos bei Patienten, die für eine größere Operation vorgesehen sind; der Beurteilung des Koagulationsrisikoprofils bei Patienten mit hohem Risiko von kardiovaskulärer Krankheit; der Beurteilung des Koagulationrisikos bei Patienten mit periphärer vaskulärer Krankheit; oder der Untersuchung des Profils von Patienten mit Blutungsstörungen.

11. Ein Apparat zur Bestimmung des Vorliegens oder des Risikos zur Entwicklung eines Zustands oder Fehlfunktion, die mit zellulären Abnormalitäten verknüpft ist, in einem oder mehreren Individuen, umfassend:
i. ein Kontrollgerät;
ii. eine Vielzahl von Mikrokapillarröhrchen, jedes mit einem adhäsiven Substrat, das auf deren inneren Oberfläche immobilisiert ist, versehen;
iii. eine Pumpe, die durch das Kontrollgerät kontrolliert wird, zum Führen einer biologischen Probe von dem oder den Individuen durch jede der Mikrokapillarröhrchen in einer laminaren Strömung über das adhäsive Substrat unter definierten Strömungsbedingungen und für eine Zeit, die dazu ausreicht, die Zellen der biologischen Probe dazu zu befähigen, sich an das adhäsive Substrat zu binden; und
iv. Detektionsmittel, die durch das Kontrollgerät kontrolliert werden, zur Bestimmung der Anzahl der Zellen, die an das adhäsive Substrat in jeder der Mikrokapillarröhrchen gebunden sind, durch Zerstören der gebundenen Zellen und anschließendem Detektieren eines Markers auf der Zelloberfläche oder in der Membran oder dem Zytosol der Zellen; worin die Detektionsmittel ein Spektrometer umfassen.

12. Ein Apparat nach Anspruch 11, weiterhin umfassend:
v. Mittel zum Vergleichen der ermittelten Bindungskapazität der Zellen mit einer zuvor bestimmten Standardbindungskapazität für solche Zellen.

13. Ein Apparat nach Anspruch 11, worin das adhäsive Substrat Plättchen oder deren Vorgänger bindet und ausgewählt ist aus der Gruppe, bestehend aus vWf, Fibrinogen, Kollagen, Vitronectin, Laminin oder Fibronectin.

14. Ein Verfahren zur Bestimmung des Modulationeffektes einer Substanz auf die Bindungskapazität von Zellen in einer biologischen Probe eines Individuums, umfassend:
i. Führen der biologischen Probe in Gegenwart dieser Substanz in einer laminaren Strömung über ein adhäsives Substrat, das auf der inneren Oberfläche eines Mikrokapillarröhrchens immobilisiert ist, unter definierten Strömungsbedingungen und eine Zeitdauer, die es den Zellen der biologischen Probe ermöglicht, an das adhäsive Substrat zu binden, und anschließendem Bestimmen der Anzahl der Zellen, die an das adhäsive Substrat gebunden sind durch Zerstören der gebundenen Zellen und anschließendem Detektieren eines Markers auf der Zelloberfläche oder in der Membran oder dem Zytosol dieser Zellen, um die Bindungskapazität dieser Zellen in Gegenwart dieser Substanz anzugeben; und
ii. Vergleichen des in Verfahrensschritt (i) erhaltenen Resultates mit dem Resultat, das erhalten wird, wenn der Verfahrensschritt (i) in Abwesenheit dieser Substanz durchgeführt wird.

15. Ein Diagnoseverfahren zur Bestimmung des Vorliegens oder des Risikos eines Zustands oder Funktionsstörung, die mit zellulären Abnormalitäten verknüpft ist, bei einem oder mehreren Individuen, umfassend:
i. Bestimmen der Bindungskapazität von Zellen in einer Vielzahl von biologischen Proben von einem oder mehreren Individuen in einem System umfassend:
(a) eine Kontrollvorrichtung;
(b) eine Vielzahl von Mikrokapillarröhrchen, jedes mit einem adhäsiven Substrat, das auf dessen inneren Oberfläche immobilisiert ist, versehen;
(c) eine Pumpe, die von dem Kontrollgerät kontrolliert wird, zum Führen einer biologischen Probe durch jedes dieser Mikrokapillarröhrchen in einer laminaren Strömung über das adhäsive Substrat unter definierten Strömungsbedingungen und einer Zeitdauer, die es den Zellen der biologischen Probe ermöglicht, an das adhäsive Substrat zu binden; und
(d) Detektionsmittel, die von dem Kontrollgerät kontrolliert werden, um die Anzahl der Zellen, die an das adhäsive Substrat in jeder der Mikrokapillarröhrchen gebunden sind, durch Zerstören der gebundenen Zellen und anschließendem Detektieren eines Markers auf der Zelloberfläche oder in der Membran oder dem Zytosol dieser Zellen zu bestimmen; und
ii. Vergleichen der ermittelten Bindungskapazität dieser Zellen in jeder biologischen Probe mit einer zuvor bestimmten Standardbindungskapazität für solche Zellen, worin die Variation der ermittelten Bindungskapazität von der zuvor bestimmten Standardbindungskapazität auf das Vorliegen oder das Risiko zum Entwickeln eines Zustands oder einer Fehlfunktion, die mit zellulären Abnormalitäten verknüpft ist, bei dem Individuum, das die biologische Probe stellt, hinweist.

16. Ein Verfahren nach Anspruch 15, worin das System weiterhin umfasst:
(e) Mittel zum Vergleichen der ermittelten Bindungskapazität dieser Zellen in jeder biologischen Probe mit einer zuvor bestimmten Standardbindungskapazität für solche Zellen.

17. Ein Diagnoseverfahren nach Anspruch 15, worin die biologischen Proben ausgewählt sind aus der Gruppe, bestehend aus natürlichem Vollblut, nicht gerinnbaren Blut, Plasma, Plättchen, Plättchen, die gewaschen und in isotonischem Puffer resuspendiert wurden, Lymphe, weißen Blutkörperchen, roten Blutkörperchen, Gewebextrakt oder Biopsiematerial.

18. Ein Diagnoseverfahren nach Anspruch 15, worin die Zellen Plättchen oder deren Vorgänger umfassen.

19. Ein Diagnoseverfahren nach Anspruch 15, worin das adhäsive Substrat ausgewählt ist aus der Gruppe, bestehend aus Rezeptoren für Zelloberflächenliganden, Liganden für Zelloberflächenrezeptoren und Immunoglobulin-Molekülen, die dazu befähigt sind mit Zelloberflächenantigenen oder Epitopen in Wechselwirkung zu treten, immobilisierten Zellen, die mit Zellen in der Probe in Interaktion treten können.

20. Ein Diagnoseverfahren nach Anspruch 19, worin das adhäsive Substrat Plättchen oder deren Vorgänger bindet, und ausgewählt ist aus der Gruppe, bestehend aus vWf, Fibrinogen, Kollagen, Vitronectin, Laminin oder Fibronectin.

21. Ein Diagnoseverfahren nach Anspruch 15, worin die definierten Strömungsbedingungen eine Strömungsrate in dem Bereich von etwa 20 zu etwa 20.000 s⁻¹, insbesondere von etwa 30 bis etwa 3.000 s⁻¹, umfassen.

22. Ein Diagnoseverfahren nach Anspruch 15, worin die Zellen Plättchen oder deren Vorgänger sind und der Marker ausgewählt ist aus Lactatdehydrogenase (LDH), Plättchenfaktor 4, P-Selektin, Thromboxan B2 oder β-Thromoboglobulin.

23. Ein Diagnoseverfahren nach Anspruch 22, worin der Marker LDH ist und über das spektrographische Mittel detektiert wird.

24. Ein Diagnoseverfahren nach Anspruch 15, in dem der Zustand oder die Funktionsstörung die Entwicklung eines Blutgerinnsels ist.

25. Ein Diagnoseverfahren nach Anspruch 15, worin dieses Verfahren verwendet wird zur Beurteilung von kardiovaskulärem Risiko in ansonsten gesunden Individuen; zur Beurteilung von Patienten, die einen thrombotischen Zustand erlitten hatten; zur Überwachung der Wirksamkeit der zuvor beschriebenen Anti-Plättchen-Therapie; der Beurteilung des Blutungs- oder Koagulationsrisikos bei Patienten, die für eine größere Operation vorgesehen sind; der Beurteilung des Koagulationsrisikoprofils bei Patienten mit hohem Risiko von kardiovaskulären Krankheiten; der Einschätzung des Koagulationsrisikos bei Patienten mit periphären vaskulären Krankheiten; oder der Untersuchung des Profils von Patienten mit Blutungsstörungen.

## Revendications

1. Procédé diagnostique pour la détection chez un sujet de la présence ou du risque de développement d'un état ou trouble impliquant des anomalies cellulaires, comprenant :
i. la détermination du pouvoir de liaison de cellules dans un échantillon biologique provenant du sujet par passage dudit échantillon biologique suivant un écoulement laminaire sur un substrat adhésif immobilisé sur la surface interne d'un tube microcapillaire dans des conditions d'écoulement définies et pour une durée suffisante pour permettre la liaison des cellules contenues dans l'échantillon biologique au substrat adhésif, et la détermination ensuite du nombre desdites cellules liées au substrat adhésif par dissociation des cellules liées puis détection d'un marqueur présent à la surface cellulaire ou dans la membrane ou le cytosol desdites cellules en tant qu'indicateur du pouvoir de liaison desdites cellules; et
ii. la comparaison du pouvoir de liaison déterminé desdites cellules contenues dans l'échantillon biologique au pouvoir de liaison étalon prédéterminé de telles cellules, dans lequel une variation du pouvoir de liaison déterminé par rapport au pouvoir de liaison étalon prédéterminé est un indicateur de la présence ou du risque de développement d'un état ou trouble impliquant des anomalies cellulaires chez le sujet.

2. Procédé diagnostique selon la revendication 1, dans lequel l'échantillon biologique est choisi dans le groupe consistant en le sang natif total, le sang total additionné d'anticoagulant, le plasma, les plaquettes, les plaquettes lavées et remises en suspension dans du tampon isotonique, la lymphe, les globules blancs, les globules rouges, un extrait tissulaire ou un spécimen de biopsie.

3. Procédé diagnostique selon la revendication 1, dans lequel les cellules comprennent des plaquettes ou des précurseurs de celles-ci.

4. Procédé diagnostique selon la revendication 1, dans lequel le substrat adhésif est choisi dans le groupe consistant en des récepteurs de ligands de surface cellulaires, des ligands de récepteurs de surface cellulaire, des molécules d'immunoglobulines capables d'interagir avec les antigènes ou épitopes de surface cellulaire, des cellules immobilisées ayant le pouvoir d'interagir avec des cellules dans l'échantillon.

5. Procédé diagnostique selon la revendication 4, dans lequel le substrat adhésif lie les plaquettes ou les précurseurs de celles-ci et est choisi dans le groupe consistant en vWf, le fibrinogène, le collagène, la vitronectine, la laminine ou la fibronectine.

6. Procédé diagnostique selon la revendication 1, dans lequel les conditions d'écoulement définies comprennent une vitesse de cisaillement comprise dans l'intervalle d'environ 20 à environ 20000 s⁻¹, plus particulièrement d'environ 30 à 3000 s⁻¹.

7. Procédé diagnostique selon la revendication 1, dans lequel les cellules sont des plaquettes ou des précurseurs de celles-ci, et le marqueur est choisi parmi la lactate-deshydrogénase (LDH), le facteur plaquettaire 4, la P-sélectine, le thromboxane B2 ou la β-thromboglobuline.

8. Procédé diagnostique selon la revendication 7, dans lequel le marqueur est LDH et est détecté par des moyens spectrographiques.

9. Procédé diagnostique selon la revendication 1, dans lequel l'état ou trouble est la formation d'un caillot sanguin.

10. Procédé diagnostique selon la revendication 1, dans lequel ledit procédé est utilisé dans le dressage d'un bilan complet du risque cardiovasculaire chez des sujets autrement en bonne santé, l'évaluation de patients ayant déjà subi un évènement thrombotique, le suivi de l'efficacité d'un traitement antiplaquettaire déjà prescrit, l'évaluation du risque thrombotique ou hémorragique chez des patients pour lesquels une opération chirurgicale majeure est programmée, l'évaluation du profil de risque thrombotique chez des patients hautement prédisposés à une maladie cardiovasculaire, l'évaluation du profil de risque thrombotique chez des patients atteints de maladie vasculaire périphérique, ou l'analyse du profil de patients atteints de troubles hémorragiques.

11. Appareil pour la détection chez un ou plusieurs sujets de la présence ou d'un risque de développement d'un état ou trouble impliquant des anomalies cellulaires comprenant :
*i*. un dispositif de commande;
*ii*. une pluralité de tubes microcapillaires, ayant chacun un substrat adhésif immobilisé sur la surface interne du tube;
*iii*. une pompe commandée par ledit dispositif de commande pour le passage d'un échantillon biologique provenant du ou de chacun des sujets à travers chacun desdits tubes microcapillaires suivant un écoulement laminaire sur ledit substrat adhésif dans des conditions d'écoulement définies et pour une durée suffisante pour permettre la liaison des cellules issues de l'échantillon biologique au substrat adhésif; et
*iv.* des moyens de détection commandés par ledit dispositif de commande pour déterminer le nombre desdites cellules liées au substrat dans chacun desdits tubes microcapillaires par dissociation des cellules liées et détection ensuite d'un marqueur présent à la surface cellulaire ou dans la membrane ou le cytosol desdites cellules;
dans lequel lesdits moyens de détection comprennent un spectrophotomètre.

12. Appareil selon la revendication 11 comprenant en outre
*v.* des moyens de comparaison du pouvoir de liaison déterminé desdites cellules au pouvoir de liaison étalon prédéterminé de telles cellules.

13. Appareil selon la revendication 11, dans lequel le substrat adhésif lie les plaquettes ou précurseurs de celles-ci et est choisi dans le groupe consistant en vWF, le fibrinogène, le collagène, la vitronectine, la laminine ou la fibronectine.

14. Procédé de détermination de l'effet modulateur d'une substance sur le pouvoir de liaison des cellules dans un échantillon biologique provenant d'un sujet comprenant :
i. le passage dudit échantillon biologique en présence de ladite substance suivant un écoulement laminaire sur un substrat adhésif immobilisé sur la surface interne d'un tube microcapillaire dans des conditions d'écoulement définies et pour une durée suffisante pour permettre la liaison des cellules issues de l'échantillon biologique au substrat adhésif, et la détermination ensuite du nombre desdites cellules liées au substrat adhésif par dissociation des cellules liées puis détection d'un marqueur présent à la surface cellulaire ou dans la membrane ou le cytosol desdites cellules en tant qu'indicateur du pouvoir de liaision desdites cellules en présence de ladite substance; et
iii. la comparaison du résultat obtenu à l'étape (i) au résultat obtenu en réalisant l'étape (i) en l'absence de ladite substance

15. Procédé diagnostique pour la détection chez un ou plusieurs sujets de la présence ou du risque de développement d'un état ou trouble impliquant des anomalies cellulaires, comprenant :
i. la détermination du pouvoir de liaison des cellules contenues dans une pluralité d'échantillons biologiques provenant du ou de chacun des sujets dans un système comprenant :
(a) un dispositif de commande;
(b) une pluralité de tubes microcapillaires, ayant chacun un substrat adhésif immobilisé sur la surface interne du tube;
(c) une pompe commandée par ledit dispositif de commande pour le passage d'un échantillon biologique à travers chacun desdits tubes microcapillaires suivant un écoulement laminaire sur ledit substrat adhésif dans des conditions d'écoulement définies et pour une durée suffisante pour permettre la liaison des cellules issues de l'échantillon biologique au substrat adhésif; et
(d) des moyens de détection commandés par ledit dispositif de commande pour déterminer le nombre desdites cellules liée au substrat dans chacun desdits tubes microcapillaires par dissociation des cellules liées et détection ensuite d'un marqueur présent à la surface cellulaire ou dans la membrane ou le cytosol desdites cellules; et
ii. la comparaison du pouvoir d'adhérence déterminé desdites cellules dans chaque échantillon biologique au pouvoir de liaison étalon prédéterminé de telles cellules, dans lequel une variation du pouvoir d'adhérence déterminé par rapport au pouvoir de liaison étalon prédéterminé est un indicateur de la présence ou du risque de développement d'un état ou trouble impliquant des anomalies cellulaires chez le sujet dont provient ledit échantillon biologique.

16. Procédé selon la revendication 15, dans lequel ledit système comprend en outre
(e) des moyens de comparaison du pouvoir de liaison déterminé desdites cellules dans chaque échantillon biologique au pouvoir de liaison étalon prédéterminé de telles cellules.

17. Procédé diagnostique selon la revendication 15, dans lequel les échantillons biologiques sont choisis dans le groupe consistant en le sang natif total, le sang total additionné d'anticoagulant, le plasma, les plaquettes, les plaquettes lavées et remises en suspension dans du tampon isotonique, la lymphe, les globules blancs, les globules rouges, un extrait tissulaire ou un spécimen de biopsie

18. Procédé diagnostique selon la revendication 15, dans lequel les cellules comprennent des plaquettes ou des précurseurs de celles-ci.

19. Procédé diagnostique selon la revendication 15, dans lequel le substrat adhésif est choisi dans le groupe consistant en des récepteurs de ligands de surface cellulaires, des ligands de récepteurs de surface cellulaire, des molécules d'immunoglobulines capables d'interagir avec les antigènes ou épitopes de surface cellulaire, des cellules immobilisées ayant le pouvoir d'interagir avec des cellules dans l'échantillon.

20. Procédé diagnostique selon la revendication 19, dans lequel le substrat adhésif lie les plaquettes ou les précurseurs de celles-ci et est choisi dans le groupe consistant en vWf, le fibrinogène, le collagène, la vitronectine, la laminine ou la fibronectine.

21. Procédé diagnostique selon la revendication 15, dans lequel les conditions d'écoulement définies comprennent une vitesse de cisaillement comprise dans l'intervalle d'environ 20 à environ 20000 s⁻¹, plus particulièrement d'environ 30 à 3000 s⁻¹.

22. Procédé diagnostique selon la revendication 15, dans lequel les cellules sont des plaquettes ou des précurseurs de celles-ci, et le marqueur est choisi parmi la lactate-deshydrogénase (LDH), le facteur plaquettaire 4, la P-sélectine, le thromboxane B2 ou la β-thromboglobuline.

23. Procédé diagnostique selon la revendication 22, dans lequel le marqueur est LDH et est détecté par des moyens spectrographiques.

24. Procédé diagnostique selon la revendication 15, dans lequel l'état ou trouble est la formation d'un caillot sanguin.

25. Procédé diagnostique selon la revendication 15, dans lequel ledit procédé est utilisé dans le dressage d'un bilan complet du risque cardiovasculaire chez des sujets autrement en bonne santé, l'évaluation de patients ayant déjà subi un évènement thrombotique, le suivi de l'efficacité d'un traitement antiplaquettaire déjà prescrit, l'évaluation du risque thrombotique ou hémorragique chez des patients pour lesquels une opération chirurgicale majeure est programmée, l'évaluation du profil de risque hémorragique chez des patients hautement prédisposés à une maladie cardiovasculaire, l'évaluation du profil de risque thrombotique chez des patients atteints de maladies vasculaires périphériques, ou l'analyse du profil de patients atteints de troubles hémorragiques.
